# EUROPEAN PATENT APPLICATION

(11) **EP 2 165 998 A1**
(43) Date of publication of application: **24.03.2010**
(21) Application number: 09156196.9
(22) Date of filing: 25.03.2009
(51) Int. Cl.: C07C 51/493, C07C 67/48, C07C 67/60, C11C 3/00

(54) **Method for selective esterification of free fatty acids in triglycerides**

(30) Priority: 15.09.2008 US 192085 P
(71) Applicant: Rohm and Haas Company, Philadelphia, PA 19106-2399 (US)
(72) Inventor: Banavali, Rajiv, Manohar, Rydal, PA 19046 (US); Schultz, Alfred, Karl, Maple Glen, PA 19002 (US)
(74) Representative: Kent, Venetia Katherine

(57) **Abstract**

A method for the selective esterification of free fatty acids, alone or in triglycerides, with C₁-C₈ aliphatic alcohols or diols. The method uses a selective heterogeneous esterification catalyst. The catalyst is contacted with a reaction mixture containing a triglyceride having at least 0.5% free fatty acids, or a reaction mixture containing only free fatty acids, and a C₁-C₈ aliphatic alcohol or diol under conditions suitable for esterification.

## Description

This invention relates generally to a method for the selective esterification of free fatty acids, alone or in triglycerides, with alcohols to produce fatty acid alkyl esters.

Fatty acids are of tremendous importance in diet. Dietary fat sources are composed of a variety of complex mixtures of such fatty acids. Saturated fatty acids are considered negative or harmful, while certain monosaturated ones are considered beneficial. While cis acids are better, trans acids are unfavorable as they correlate with circulatory diseases such as atherosclerosis and coronary heart disease. Similarly, Omega-3 (double bond at 3^{rd} carbon) fatty acids are considered better than Omega-6 fatty acids. Often these fatty acids are present together in a given material and a need therefore exists to separate and purify the beneficial or "good" acids from the negative or "bad" ones.

Typically, purification of fatty acids is achieved by techniques that are cumbersome, expensive, and generally done in a laboratory environment. For example, purification by Thin Layer Chromotography, GC, or High Performance Liquid Chromatography columns, and urea fractionation is typical. Separation by counter-current chromatography is used for the preparation of highly unsaturated fatty acids on a semi-preparative scale. Separation of unsaturated fatty acids is also done by silver nitrate impregnated silica gel columns.

Especially with respect to saturated fatty acids conventional methods often prove to be inefficient. Specifically referring to urea fractionation, upon crystallization, urea forms inclusion complexes with some long-chain aliphatic compounds. Saturated fatty acids form complexes readily as trans fatty acids, their formation being less efficient with increasing number of double bonds or in the presence of branched chains. This procedure cannot be used as an analytical technique but is frequently applied to obtain a concentrate of polyunsaturated or branched-chain fatty acids. Thus a need exists for a simpler, more efficient process for separating free fatty acids.

The present invention solves this need by providing a much simpler and efficient process for separation and purification of specific free fatty acids. The invention comprises reacting a mixture of free fatty acids, either as neat or in the corresponding oil and fat, with alcohol via selective esterification by a catalyst that selectively esterifies the desired free fatty acid(s). After the selective esterification, the remaining free fatty acid(s) and the ester(s) can be easily separated by fractional distillation and/or liquid/liquid extraction.

The present invention is directed to a method for the selective esterification of free fatty acids in triglycerides with a C₁-C₈ aliphatic alcohol or diol; said method comprising steps of contacting a selective heterogeneous esterification catalyst with a reaction mixture comprising a C₁-C₈ aliphatic alcohol or diol and a mixture comprising 0-99.5% triglycerides and 0.5-100% free fatty acids, under conditions suitable for esterification, to produce a product stream; wherein the 0.5 - 100% free fatty acids comprises a mixture of at least two free fatty acids and; further wherein the product stream comprises at least one ester of a free fatty acid and at least one unreacted free fatty acid.

All percentages are weight percentages, and all temperatures are in °C, unless otherwise indicated. Weight percentages of ion exchange resin are based on dry resin. An "alkyl" group is a saturated hydrocarbyl group having from one to twenty carbon atoms in a linear, branched or cyclic arrangement. In one preferred embodiment, alkyl groups are acyclic. "Triglycerides" used in this invention are fats or oils comprising glycerine triesters of fatty acids. Preferably, triglycerides are in the form of vegetable oils, but animal fats can also be used as a starting material. Fatty acids are acyclic aliphatic carboxylic acids containing from 8 to 22 carbon atoms; typically, they contain from 12 to 22 carbon atoms. With respect to carbon-carbon bonds, the fatty acids may be saturated, monounsaturated or polyunsaturated (typically 2 or 3 carbon-carbon double bonds). Natural fats may also contain small amounts of other esterified, or free fatty acids, as well as small amounts (1-4%) of phospholipids, e.g., lecithin, and very small amounts (<1%) of other compounds, e.g., tocopherols.. A "reaction zone" is a flow reactor or a portion of a flow reactor. When a single flow reactor is used, the zones are divided from each other by points along the reactor at which water is separated from the reaction mixture. When multiple reactors are used, with separation of water between reactors, typically each reactor is a reaction zone. Suitable reactors include, e.g., packed-bed reactors, continuous stirred tank reactors, column reactors, etc. A reaction zone may encompass multiple stages in a column reactor. Preferably, reactors are configured as cocurrent flow reactors, i.e., the fatty acid and alcohol pass through the reactor in the same direction. Typically, the product stream from the reaction zone is sent to a transesterification process, where it is contacted with a transesterification catalyst and an alcohol, preferably after separating water.

In some embodiments of the invention, the reaction mixture is heated in a temperature range from 40°C to 160°C for at least 15 minutes in contact with the selective esterification catalyst. Alternatively, the temperature is at least 50°C, alternatively at least 55°C, alternatively at least 60°C, alternatively at least 70°C. Alternatively, the temperature is no greater than 120°C, alternatively no greater than 110°C, alternatively no greater than 90°C, alternatively no greater than 85°C, alternatively no greater than 80°C, alternatively no greater than 75°C. Typically, the reaction is carried out in a flow reactor, and preferably the contact time is at least 30 minutes, alternatively at least 45 minutes.

Preferably, the contact time is no more than 6 hours, alternatively no more than 4 hours, alternatively no more than 2 hours. In some embodiments of the invention in which the alcohol is methanol, the reaction is carried out at 70°C to 110°C under pressure, alternatively from 75°C to 100°C.

In some embodiments of the invention, the mixture contains 0% to 99% triglycerides and 1% to 100% free fatty acids. In some embodiments of the invention, the mixture contains 1% to 99% triglyceride and from 1% to 99% free (unesterified) fatty acids. In some embodiments of the invention, the mixture contains a triglyceride and no more than 80% free fatty acids, alternatively no more than 50%, alternatively no more than 40%, alternatively no more than 30%, alternatively no more than 20%, alternatively no more than 15%, alternatively no more than 10%. In some embodiments, the mixture contains a triglyceride and at least 1% free fatty acids, alternatively at least 2%, alternatively at least 3%, alternatively at least 4%, alternatively at least 5%. In some embodiments of the invention, the mixture contains 60% to 98% triglyceride and from 2% to 40% free fatty acids. In all cases the mixture contains at least two free fatty acid compounds. Post-esterification, at least one of the free fatty acids is converted to an ester of the acid and at least on free fatty acid remains unreacted.

In some embodiments of the invention, the C₁-C₈ aliphatic alcohol or diol is a C₁-C₄ alcohol; alternatively it is methanol, ethanol or n-butanol; alternatively it is methanol or ethanol; and most preferably methanol. In some embodiments of the invention, the C₁-C₈ aliphatic alcohol or diol is a C₁-C₈ diol, alternatively a C₁-C₄ diol, e.g., ethylene glycol. In some embodiments of the invention, the alcohol is present in an amount of at least 1.1 equivalents based on free fatty acid, alternatively at least 2 equivalents, alternatively at least 5 equivalents, alternatively at least 10 equivalents, alternatively at least 15 equivalents. In some embodiments of the invention, the alcohol is present in an amount of no more than 25 equivalents.

Suitable heterogeneous selective esterification catalysts include, e.g., acidic ion exchange resins (e.g., a strong cation exchange resin in the hydrogen form), heterogeneous tin-containing catalysts and combinations thereof. The catalyst used in each reaction zone may be the same or different.

In some embodiments of the invention, the heterogeneous esterification catalyst is a gel-type acidic ion exchange resin having 0.25% to 2.75% crosslinker. In these embodiments, the resin is not a macroreticular resin, which is a resin having a surface area from 25 m²/g to 200 m²/g and an average pore diameter from 50 Å to 500 Å; alternatively a surface area from 30 m²/g to 80 m²/g and an average pore diameter from 100 Å to 300 Å. Suitable gel-type resins include, e.g., acrylic resins, styrenic resins, and combinations thereof. Resins contain polymerized units of a multiethylenically unsaturated monomer (crosslinker). The level of crosslinker in the resin is no more than 4% alternatively no more than 2.5%, alternatively no more than 2.25%, alternatively no more than 2%, alternatively no more than 1.75%. In some embodiments, the level of crosslinker is at least 0.5%, alternatively at least 0.75%, alternatively at least 1%. Preferably, the average particle size of the gel resin is from 100 µm to 2000 µm, more preferably from 200 µm to 800 µm. In some embodiments of the invention, the ion exchange resin comprises polymerized units of styrene and a crosslinker, e.g., divinyl aromatics; di-, tri- and tetra-(meth)acrylates or (meth)acrylamides; di-, tri- and tetra-allyl ethers and esters; polyallyl and polyvinyl ethers of glycols and polyols. In some embodiments of the invention, the crosslinker is diethylenically unsaturated, e.g., divinylbenzene (DVB). In some embodiments of the invention, the acid functionality of the ion exchange resin comprises sulfonic acid groups, carboxylic acid groups, phosphoric acid groups or a mixture thereof. A typical acidic ion exchange resin has from 0.4 to 8 meq/g acid functionality, on a dry basis, alternatively at least 2 meq/g, alternatively at least 4 meq/g. Preferably, the acid functionality is in the form of sulfonic acid groups.

### Examples

### Example 1: Selective Esterification; Comparing fatty acids with differing chain lengths

In a four-neck 1L RB flask equipped with a Soxhlet condenser containing 50 g activated molecular sieves 3A, thermometer and mechanical stirrer, was added dry polymeric catalyst (13.75 g, 5% by weight of reaction mixture). Canola oil (202.5 g, 0.23 moles triglycerides) was charged to the flask and mechanical stirring started at 185 RPM. Then, erucic acid (37.9g, 0.112 moles) and lauric acid (22.4 g, 0.112 moles) were added and the flask was heated by external infrared lamp to reach 60 C over 20 minutes. At 60 C, methanol (4 g, 0.13 mole or 1.1 equivalent of lauric acid) was charged to the flask. The mixture was allowed to reach reflux temperature (∼65-67°C) with efficient stirring (235 rpm). The reflux was condensed through a water condenser and passed through the molecular sieves back into the flask.

The reaction was carried out at 65°C-67°C (reflux temperature) and atmospheric pressure for 30 min. After 30 min., the mixture was cooled to ambient temperature. The catalyst was recovered by filtration from the organic phase. Conversion of specific acids is summarized in Table 1

### EXAMPLE 2: Selective Esterification; Comparing fatty acids with differing amounts of unsaturation.

Using the procedure listed in example 1, linoleic acid (31.4 g, 0.112 moles) and stearic acid (31.8 g, 0.112 moles) were reacted with methanol. Results are summarized in table 1.

### EXAMPLE 3: Selective Esterification; Comparing fatty acids with differing amounts of unsaturation

Using the procedure listed in example 1, linoleic acid (31.4 g, 0.112 moles) and oleic acid (31.6 g, 0.112 moles) were reacted with methanol. Results are summarized in table 1.

### EXAMPLE 4: Selective Esterification; Comparing fatty acids with differing chain lengths

Using the procedure listed in example 1, palmitic acid (28.7 g, 0.112 moles) and stearic acid (31.8 g, 0.112 moles) were reacted with methanol. Results are summarized in table 1.

| Table 1. Results Showing Fatty Acid Selectivity. | | |
|---|---|---|
| Example | Fatty Acid | Relative Conversion |
| 1 | Lauric Acid | 65 |
| (Comparing fatty acids with differing chain lengths) | Erucic Acid | 35 |
| 2 | Linoleic Acid | 59 |
| (Comparing fatty acids with differing amounts of unsaturation) | Stearic Acid | 41 |
| 3 | Linoleic Acid | 55 |
| (Comparing fatty acids with differing amounts of unsaturation) | Oleic Acid | 45 |
| 4 | Palmitic Acid | 52 |
| (Comparing fatty acids with differing chain lengths) | Stearic Acid | 48 |

## Claims

1. A method for the selective esterification of free fatty acids in triglycerides with a C₁-C₈ aliphatic alcohol or diol; said method comprising steps of:
contacting a selective heterogeneous esterification catalyst with a reaction mixture comprising a C₁-C₈ aliphatic alcohol or diol and a mixture comprising 0-99.5% triglycerides and 0.5-100% free fatty acids, under conditions suitable for esterification, to produce a product stream;
wherein the 0.5 - 100% free fatty acids comprises a mixture of at least two free fatty acids and;
further wherein the product stream comprises at least one ester of a free fatty acid and at least one unreacted free fatty acid.

2. The method of claim 1 in which the C₁-C₈ aliphatic alcohol or diol is methanol or ethanol.

3. The method of claim 2 in which said mixture comprises 20 wt % to 98 wt % triglyceride and 2 wt % to 80 wt % free fatty acids.

4. The method of claim 3 in which the selective heterogeneous esterification catalyst is a gel-type acidic ion exchange resin having 0.25 wt % to 2.75 wt % crosslinker, and having sulfonic acid functionality.

5. The method of claim 4 in which the reaction mixture is in contact with the catalyst in a continuous reactor in a temperature range from 40°C to 120°C for at least 15 minutes.
